(19) **Europäisches Patentamt / European Patent Office / Office européen des brevets**

(11) **EP 3 033 999 B1**

(12) **EUROPEAN PATENT SPECIFICATION**

(45) Date of publication and mention
of the grant of the patent:
**19.04.2017 Bulletin 2017/16**

(51) Int Cl.:
***A61B 5/18*** *(2006.01)* ***A61B 5/16*** *(2006.01)*

(21) Application number: **15199835.8**

(22) Date of filing: **14.12.2015**

(54) **APPARATUS AND METHOD FOR DETERMINING THE STATE OF A DRIVER**

VORRICHTUNG UND VERFAHREN ZUR BESTIMMUNG DES ZUSTANDS EINES FAHRERS

APPAREIL ET PROCÉDÉ DE DÉTERMINATION DE L'ÉTAT D'UN CONDUCTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2014 JP 2014252945**
**15.12.2014 JP 2014252940**

(43) Date of publication of application:
**22.06.2016 Bulletin 2016/25**

(73) Proprietor: **AISIN SEIKI KABUSHIKI KAISHA Kariya-shi, Aichi 448-8650 (JP)**

(72) Inventors:
- **OHSUGA, Shin**
  **Kariya-shi,, Aichi 448-8650 (JP)**
- **KOJIMA, Shinichi**
  **Nagakute-shi,, Aichi 480-1192 (JP)**

(74) Representative: **TBK**
**Bavariaring 4-6**
**80336 München (DE)**

(56) References cited:
**JP-A- 2011 128 966    US-A1- 2011 235 919**
**US-A1- 2014 140 577**

- **TAKAHIRO ISHIKAWA ET AL: "PASSIVE DRIVER GAZE TRACKING WITH ACTIVE APPEARANCE MODELS", IEEE TRANSACTIONS ON INTELLIGENT TRANSPORTATION SYSTEMS, [Online] 1 October 2004 (2004-10-01), XP055270665, Retrieved from the Internet: URL:https://www.ri.cmu.edu/pub_files/pub4/ ishikawa_takahiro_2004_2/ishikawa_takahiro _2004_2.pdf> [retrieved on 2016-05-03]**

**Description**

TECHNICAL FIELD

This disclosure relates to a determination apparatus and a determination method.

BACKGROUND DISCUSSION

In recent years, development of a face detection technology for detecting a position and an direction of a face and a state of face parts such as eyes and a mouth included in a picked-up still image or a motion picture is in progress. For example, a vehicle has a function to detect drowsy driving and the like by performing an eye opening/closing determination from a result of detection of a driver's face and to perform a predetermined process such as alerting.

In the related art, technologies to detect an opening/closing state of driver's eyes, and in addition, to use estimated information such as the drowsy driving from a driving behavior such as vehicle movement are known (for example, see JP 2008-165348A (Reference 1)). Technologies to detect the opening/closing state of driver's eyes by using both of an eye-opening degree, which is a distance between upper and lower eyelids obtained from the result of face detection, and a shape of the driver's eyelids to detect drowsy driving or the like (for example, see JP 2004-192551A: reference 2) are also known.

However, when the driver gazes downward, the eye-opening degree, which is the distance between upper and lower eyelids, is reduced, and thus erroneous eye opening/closing determination may occur. Therefore, in order to avoid such an erroneous determination, a technology to detect that the driver is sleepy by detecting whether or not the driver gazes downward from contour shapes of the driver's eyelids is known (for example, see JP 2008-171065A (reference 3) and JP 2008-167806A: reference 4).

However, in the related art, there is a case where whether or not the driver gazes downward cannot be determined only by the eyelid shape. In the related art, the eyelid shapes on a two-dimensional image present different appearances depending on a relative angular relationship between the face and a camera. Therefore, in the related art, determining the states of the driver's face and eyes accurately is difficult.

In the related art, when detecting the opening/closing state of the driver's eyes, influence of disturbance is significant, and thus accurate determination of the opening/closing of the eyes is difficult. In addition, since the eye opening/closing determination is performed on the two-dimensional image, appearance of the eyelid shape on the two-dimensional image is changed depending on the relative angular relationship between the face and the camera, and thus accurate eye opening/closing determination is difficult.

The US 2011/235919 A1 discloses obtaining a facial image acquired by picking up an image of a person subjected to image pickup from a camera, and estimating a facial expression of the person subjected to image pickup based on the obtained facial image. Next, an eye open level of the person subjected to image pickup is obtained based on the facial image, and a threshold of the eye open level is set based on the estimated facial expression of the person subjected to image pickup. When the eye open level exceeds the threshold, it is determined that the eye of the person subjected to image pickup is opened. Moreover, when the eye open level does not exceed the threshold, it is determined that the eye of the person subjected to image pickup is closed.

Further relevant prior art can be found in the US 2014/140577 A1, in Takahiro Ishikawa et al.: Passive Driver Gaze Tracking With Active Appearance Models", IEEE Transactions on Intelligent Transportation Systems, October 1, 2004, XP055270665, and in the JP 2011 128966 A.

SUMMARY

A need exists for a determination apparatus and a determination method capable of accurately determining states of driver's face and eyes.

According to the present invention, a determination apparatus and a determination method are provided as set out in the appended claims.

According to aspects of the invention, the state of the driver's face and eyes can be determined further accurately while avoiding the erroneous determination, and a state in which the driver gazes downward can be determined further accurately while avoiding the erroneous determination.

Further, according to aspects of the invention, a state that the driver closes the eyes can be determined further accurately while avoiding the erroneous determination.

Further, according to aspects of the invention, the occurrence of events in which the driver falls asleep at the wheel or drives with the eyes off the road can be prevented.

Further, according to aspects of the invention, the opening/closing state of the driver's eyes can be determined further accurately while avoiding the erroneous determination. Further, according to aspects of the invention, the state that the driver opens the eyes can be determined further accurately while avoiding the erroneous determination.

Further, according to aspects of the invention, the state in which the driver closes the eyes can be determined further accurately while avoiding the erroneous determination.

Further, according to aspects of the invention, the driver is prevented from performing drowsy driving.

BRIEF DESCRIPTION OF THE DRAWINGS

The foregoing and additional features and characteristics of this disclosure will become more apparent from the following detailed description considered with the reference to the accompanying drawings, wherein:

Fig. 1 is a perspective view illustrating a state in which part of a vehicle cabin is seen through according to an embodiment;
Fig. 2 is a drawing illustrating an example of arrangement of an image pickup device according to the embodiment;
Fig. 3 is a block diagram illustrating an example of a determination system according to the embodiment;
Fig. 4 is a block diagram illustrating a functional configuration of an ECU according to the embodiment;
Fig. 5 is a schematic drawing for explaining an example of a three-dimensional face model according to the embodiment;
Fig. 6 is a schematic drawing illustrating an example of change information registered in the three-dimensional face model according to the embodiment;
Fig. 7 is a flowchart illustrating an example of a procedure of the three-dimensional face model creating process according to the embodiment;
Fig. 8 is a flowchart illustrating an example of a procedure of a determination process according to the first embodiment;
Fig. 9 is a schematic drawing illustrating a template T created for a model M of a face in the three-dimensional face model according to the embodiment;
Fig. 10 is a block diagram illustrating a functional configuration of an ECU according to another embodiment; and

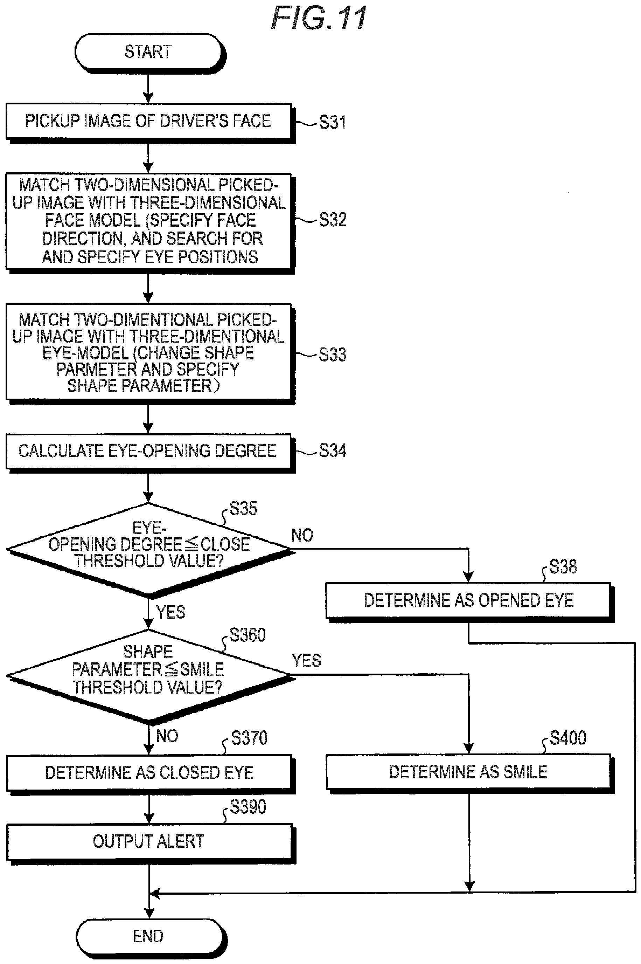

Fig. 11 is a flowchart illustrating another example of a procedure of a determination process according to the embodiment.

DETAILED DESCRIPTION

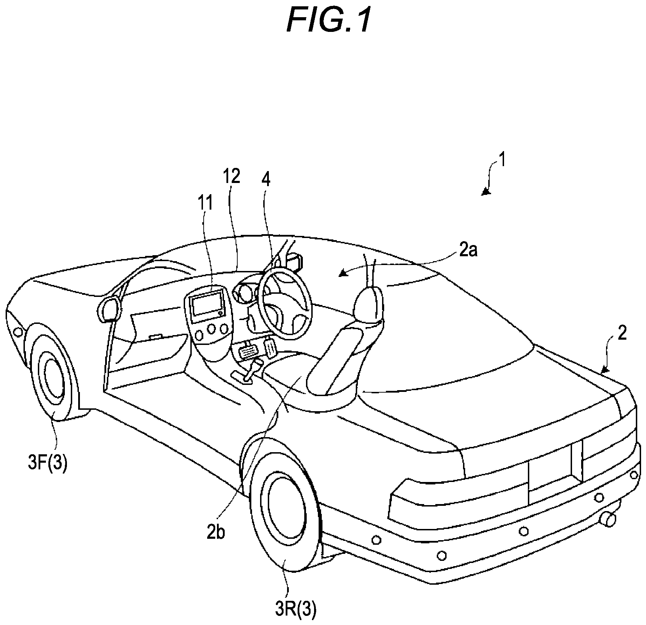

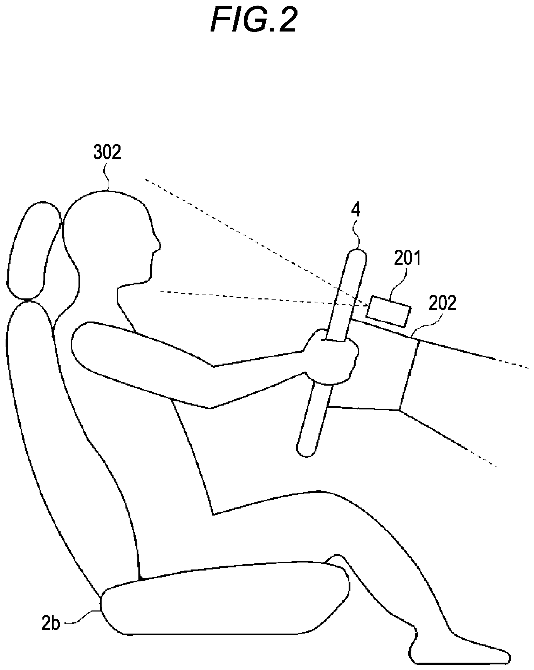

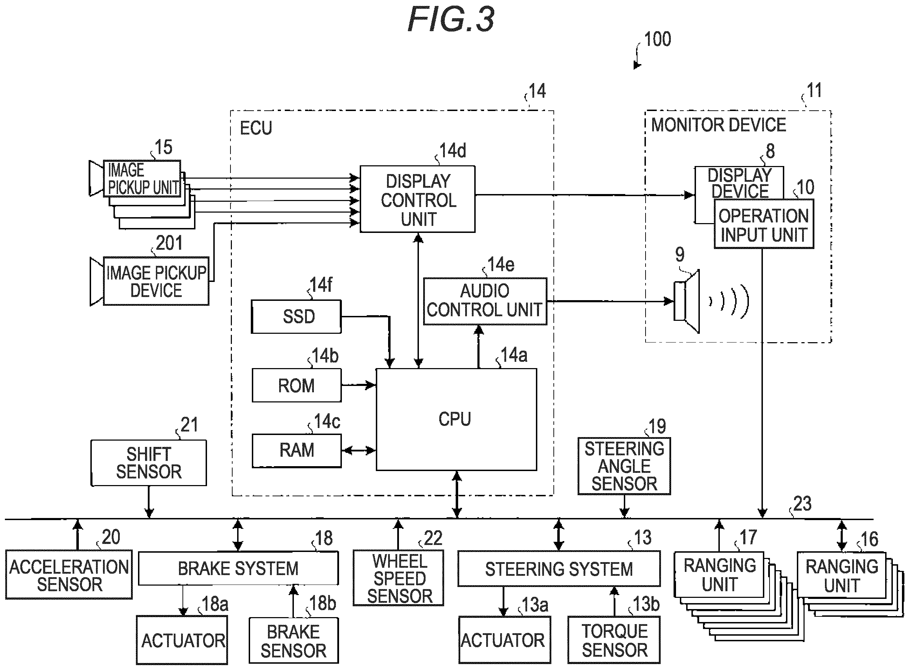

Hereinafter, an example in which a determination apparatus of an embodiment is mounted on a vehicle 1 will be described.
In the embodiment, the vehicle 1, for example, may be one of automotive vehicles employing an internal combustion engine (engine which is not illustrated) as a drive source (internal combustion engine vehicles), may be one of automotive vehicles employing an electric motor (motor which is not illustrated) as the drive source (electric automotive vehicles, fuel cell automotive vehicle, and the like), and may be one of automotive vehicles employing both of the engine and the motor as the drive source (hybrid automotive vehicles). The vehicle 1 may include various speed changers mounted thereon, and may include various apparatuses (systems, parts, and the like) required for driving the internal combustion engine or the electric motor mounted thereon. Systems, numbers, layouts, and the like of devices in the vehicle 1 relating to driving of wheels 3 may be set in various manners.
As illustrated in Fig. 1, a vehicle body 2 of the vehicle 1 constitutes a vehicle cabin 2a on which a driver (not illustrated) gets in. A steering portion 4 or the like is provided in the interior of the vehicle cabin 2a in a state of facing a driver's seat 2b as an occupant. In the present embodiment, as an example, the steering portion 4 is a steering wheel projecting from a dash board (instrument panel) 12.
As illustrated in Fig. 1, in the present embodiment, the vehicle 1 is, for example, a four-wheeler (four-wheel automotive vehicle) includes two left and right front wheels 3F and two left and right rear wheels 3R. In addition, in the present embodiment, all of the four wheels 3 are configured to be steerable.
A monitor device 11 is provided at a center portion of the dash board 12 in the interior of the vehicle cabin 2a in a vehicle width direction, that is, in a lateral direction. The monitor device 11 is provided with a display device and an audio output device. The display device is, for example, an LCD (liquid crystal display), an OELD (organic electroluminescent display), or the like. The audio output device is, for example, a speaker. The display device is covered with a transparent operation input unit such as a touch panel. The occupant can view an image displayed on a display screen of the display device via the operation input unit. The occupant can execute an operation input by manipulating an image displayed on a display screen of a display device by touching, pushing, or moving an operation input unit with fingers or the like at a corresponding position of the image.
As illustrated in Fig. 2, an image pickup device 201 is provided on a steering wheel column 202. The image pickup device 201 is, for example, a CCD (Charge Coupled Device) camera. The image pickup device 201 is adjusted in view angle and posture so that a face of a driver 302 seated on the seat 2b is positioned at a center of the field of view. The image pickup device 201 picks up images of the face of the driver 302 in sequence, and outputs image data on the images obtained by shooting in sequence.
Subsequently, a determination system having the determination apparatus in the vehicle 1 according to the embodiment will be described. Fig. 3 is a block diagram illustrating an example of a determination system 100 according to the embodiment. As illustrated in Fig. 3, the determination system 100 includes a brake system 18, a steering angle sensor 19, an acceleration sensor 20, a shift sensor 21, and a wheel speed sensor 22 as well as an ECU 14, a monitor device 11, a steering system 13, and ranging units 16 and 17, which are electrically connected via an in-vehicle network 23 as telecommunication lines. The in-vehicle network 23 is configured as, for example, CAN (Controller Area Network). The ECU 14 can control the steering system 13, and the brake system 18 by sending a control signal via the in-vehicle network 23. The ECU 14 can receive results of detection from a torque sensor 13b, a brake sensor 18b, the steering angle sensor 19, the ranging unit 16, the ranging unit 17, the acceleration sensor 20, the shift sensor 21, the wheel speed sensor 22, and an operation signal from the operation input unit via the in-vehicle network 23. The ECU 14 is an example of the determination apparatus.

The ECU 14 includes, for example, a CPU 14a (Central Processing Unit), a ROM 14b (Read Only Memory), a RAM 14c (Random Access Memory), a display control unit 14d, an audio control unit 14e, and an SSD 14f (Solid State Drive, flash memory). The CPU 14a performs control of the entire part of the vehicle 1. The CPU 14a can read out a program installed and memorized in a non-volatile memory device such as a ROM 14b and executes an arithmetic operation in accordance with the program. The RAM 14c temporarily memorizes various data used in arithmetic operation in the CPU 14a. Out of the arithmetic processing to be performed in the ECU 14, the display control unit 14d executes image processing using mainly image data obtained by an image pickup unit 15 and synthesizing of the image data displayed on the display device out of the arithmetic processing of the ECU 14. The audio control unit 14e executes mainly processing of audio data output from the audio output device out of the arithmetic processing in the ECU 14. The SSD 14f is a rewritable non-volatile memory and data can be memorized even when a power source of the ECU 14 is turned OFF. The CPU 14a, the ROM 14b, and the RAM 14c can be integrated in the same package. The ECU 14 may have a configuration in which logical arithmetic processor such as a DSP (Digital Signal Processor) or a logical circuit are used instead of the CPU 14a. Alternatively, an HDD (Hard Disk Drive) may be provided instead of the SSD 14f and SSD 14f and the HDD may be provided separately from the ECU 14.

The configuration, the arrangement, and the mode of electrical connection of the various sensors and an actuator described above are examples only, and may be set (modified) in various manners.

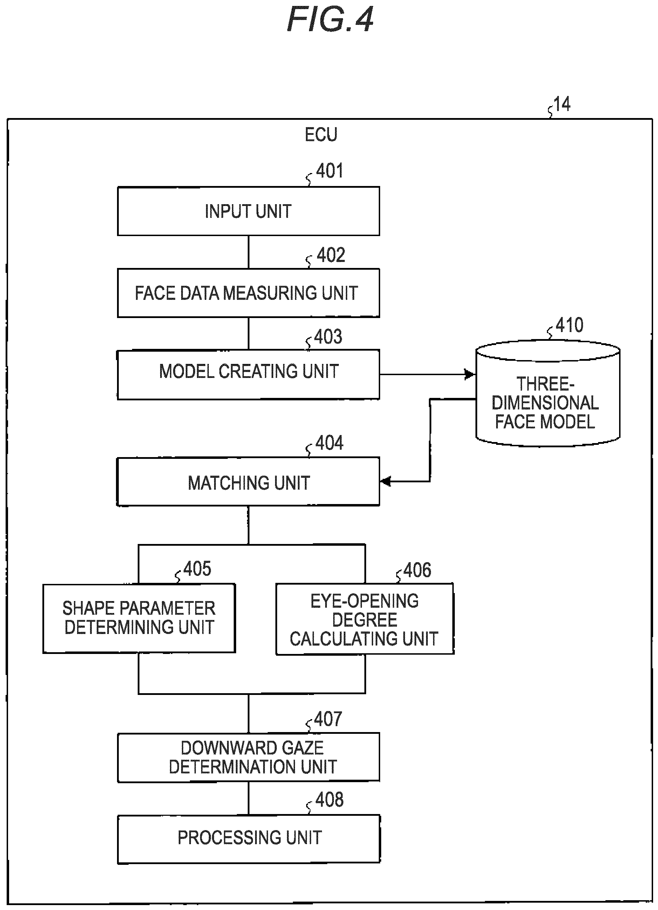

Fig. 4 is a block diagram of a functional configuration of the ECU 14 according to the embodiment. As illustrated in Fig. 4, the ECU 14 mainly includes an input unit 401, a face data measuring unit 402, a model creating unit 403, a matching unit 404, a shape parameter determination unit 405, an eye-opening degree calculating unit 406, a downward gaze determination unit 407, a processing unit 408, and a three-dimensional face model 410. Configurations of members illustrated in Fig. 4 except for the three-dimensional face model 410 are achieved by the CPU 14a configured as the ECU 14 executing the program stored in the ROM 14b. These configurations may be achieved with hardware.

The three-dimensional face model 410 is saved in a memory medium such as the SSD 14f. The three-dimensional face model 410 is a statistical face shape model, in which a three-dimensional face shape of an average test subject, positions of face parts such as eyes, a mouth, a nose, and the like of the test subject, and change information of a change in eye shape of the test subject are registered. For example, a CLM (Constrained Local Model), an AAM (Active Appearance Model), an ASM (Active Shape Model) may be used as the three-dimensional face model 410. However, the three-dimensional face model 410 is not limited to thereto.

The ECU 14 of the embodiment detects a driver's face direction and the driver's eye positions while tracking the driver's face, obtains an eye-opening degree, which is a distance between the upper and lower eyelids of the driver, specifies a shape parameter corresponding to the eye shape, and determines whether or not the driver is in a state of gazing downward (downward-gaze state) and whether or not the driver opens the eyes on the basis of the eye-opening degree and the shape parameters by using the three-dimensional face model 410. Detailed description will be given below.

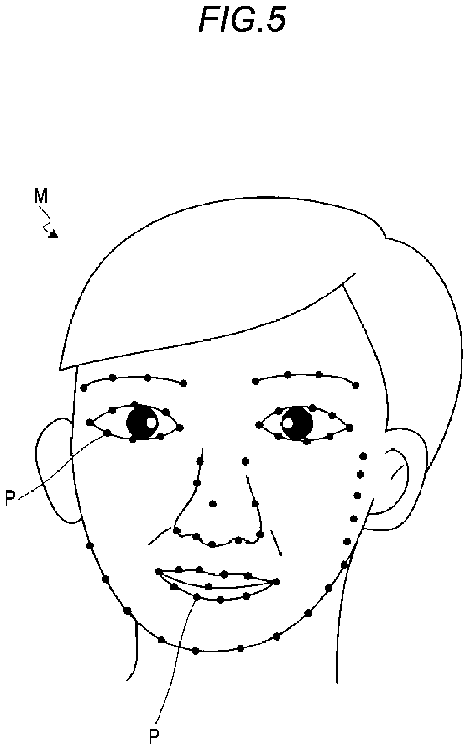

Fig. 5 is a schematic drawing for explaining an example of the three-dimensional face model 410 of the embodiment. In Fig. 5, a model M of the exemplified face is illustrated. The model M shows a three-dimensional shape of an average face from face shapes obtained from, for example, 100 test subjects, and includes multiple characteristic points P indicating predetermined face parts. The characteristic points P are expressed by a coordinate with a given point as an original point. In the example illustrated in Fig. 5, the characteristic points P expressing eyebrows, eyes, a nose, a mouth, and an outline are shown. However, the model M may include different characteristic points P from those illustrated in Fig. 5.

The change information is also registered in the three-dimensional face model 410. The change information is data that maps step-by-step change of the eye shape of the driver in association with the changes in expression of the face and actions of the driver to the shape parameters at each stage. Specifically, as the change states, a state in which the face has lack of expression and faces forward with opened eyes is defined as a standard state, and states such as step-by-step changes of the eye shape from the standard state until the expression shows a smile, step-by-step changes of the eye shape from the standard state until the eyes are closed, and step-by-step changes of the eye shape from the standard state until the eyes gaze downward (downward gaze) are mapped to the shape parameters and are registered to the three-dimensional face model 410.

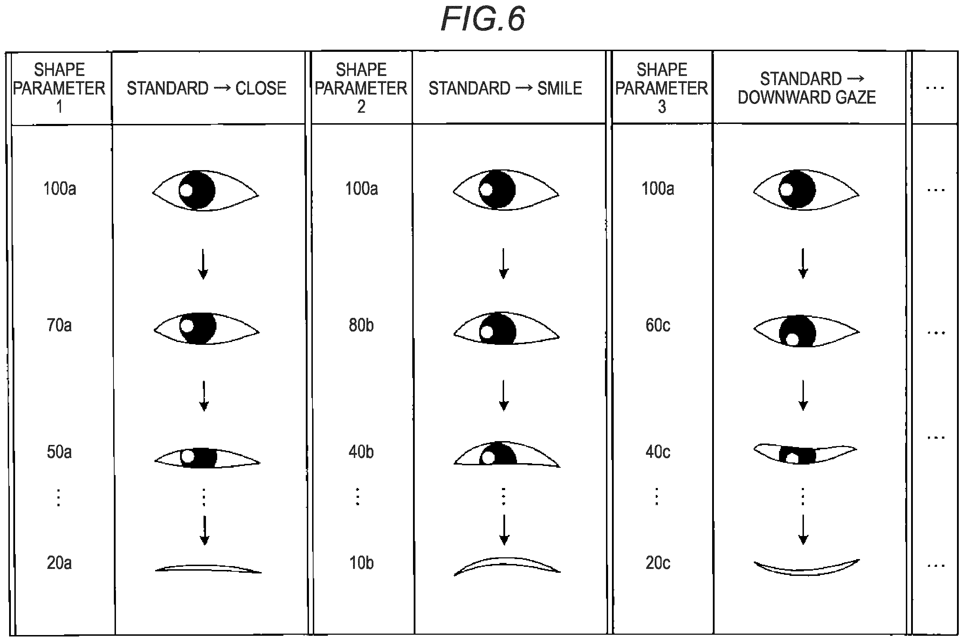

Fig. 6 is a schematic drawing illustrating an example of the change information registered in the three-dimensional face model 410 of the embodiment. An example illustrated in Fig. 6 corresponds to a shape parameter 1 in which step-by-step changes of the eye shape from the standard eye state until the eyes are closed takes different values depending on the steps. An example corresponds to a shape parameter 2 in which step-by-step changes of the eye shape from the standard eye state until the expression changes to a smile takes different values depending on the steps. An example corresponds to a shape parameter 3 in which step-by-step changes of the eye shape from the standard eye state until a downward gaze takes different values depending on the steps. All of the shape parameters 1, 2, and 3 show values reducing step-by-step as the eye shape changes from the standard eye state to the state of eye shape after the change.

However, the change of the parameters are not limited thereto, and shape parameters may be configured to change in such a manner that the value increases as the state approaches the state after the change. Alternately, the shape parameter may be configured to change differently such that the value increases and decreases depending on the type of the change.

The change in eye shape registered as the change information is not limited to the example illustrated in Fig. 6, and may be configured to map step-by-step changes of the eye shape from the standard eye state until yawning, step-by-step changes of the eye shape from the standard eye state until a state of taking a good look at something, and step-by-step changes of the eye shape from the standard eye state until a state in which the eyes are widened to the shape parameters step by step and register the same to the three-dimensional face model 410.

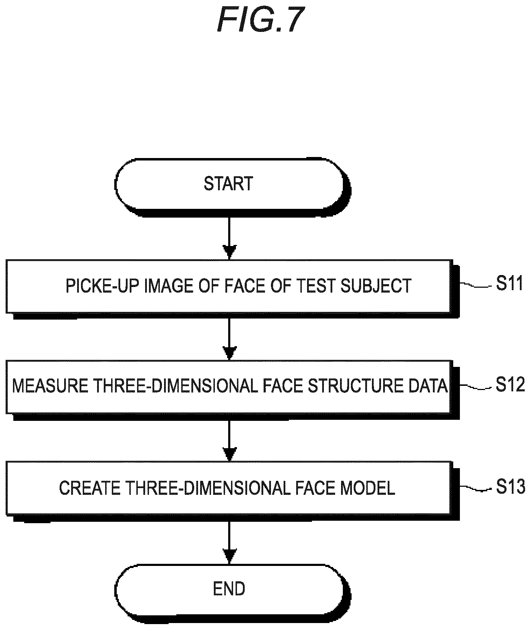

The creation of the three-dimensional face model 410 is performed by the input unit 401, the face data measuring unit 402, and the model creating unit 403 illustrated in Fig. 4 as described below. Fig. 7 is a flowchart illustrating an example of a procedure of a process of creating a three-dimensional face model 410 of the present embodiment.

A administrator or the like picks up images of one-hundred test subjects' faces, for example, by a 3D scanner or the like (S11). In S11, the test subjects are each made to perform actions of various changes such as a standard state of the face, a smile, a state in which the eyes are closed, a state of a downward gaze, and images of these states are picked up by the 3D scanner or the like. The number of the test subjects is not limited to one hundred.

The input unit 401 inputs the picked-up images described above. The face data measuring unit 402 extracts characteristic points such as face shapes and face parts including eyes or mouths of the multiple test subjects from a picked-up image input thereto, and measures three-dimensional face structure data relating to an average face shape and position of the face parts (S12). The face data measuring unit 402 is an example of the measuring unit.

In S12, the face data measuring unit 402 extracts a shape parameter corresponding to the state of the step-by-step changes of the eye shape by a statistical data analysis method such as an analysis of principal component in accordance with a procedure of creating a three-dimensional face model indicated, for example, in Non-Patent Literature "Automatic Extraction of Eye Area Structure by Active Appearance Model Search" by Go Moriyama, Takeo Kanaide, and Jeffrey F. Cohn, Shinji Ozawa, "Meeting on Image Recognition and Understanding" (MIRU 2006)" from picked-up images of variously changed actions of the test subjects which are picked up in S11.

Specifically, when expressing vectors of coordinates of points which constitute parts of the outline of the eye in the picked-up image by symbols x, the symbols x show a shape. The face data measuring unit 402 averages all of the picked-up images from the standard eye state to the state after the change and obtains an average shape xav. The face data measuring unit 402 performs the analysis of principal component on a deviation of respective xs from the standard eye state to the state after the change from xav to obtain characteristic vectors Ps. At this time, x is expressed by the expression (1).

$$x = x_{av} + P_s b_s \cdots (1)$$

Here, bs is a principal component score vector, which is referred to as a shape parameter. In other words, the face data measuring unit 402 obtains the shape parameter bs from the expression (1).

The face data measuring unit 402 maps the states of the step-by-step changes of the eye shape to the shape parameters and creates the change information illustrated in Fig. 6.

The model creating unit 403 creates an average face shape as the measured three-dimensional face structure data, the positions of the face parts, and the above-described change information as the three-dimensional face model 410 (S13). Accordingly, the model of the average face is created by being registered to the three-dimensional face model 410.

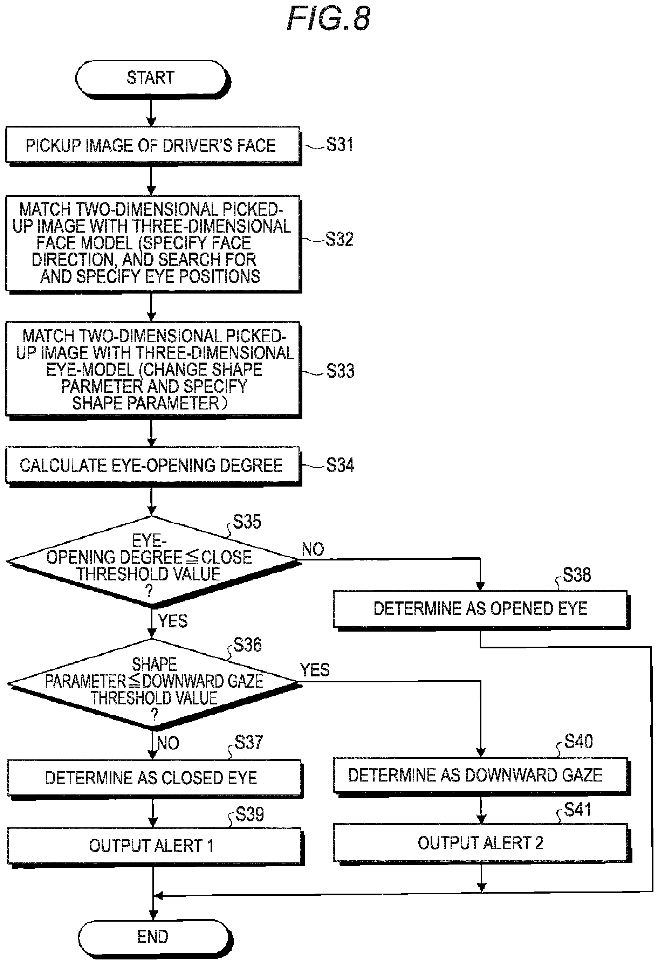

Subsequently, the determining process of the embodiment will be described. Fig. 8 is a flowchart illustrating an example of a procedure of a determination process of the present embodiment. As a first step, the image pickup device 201 picks up an image of the driver (S31). Images are picked up cyclically at regular temporal intervals. The picked-up image of the driver is input to the matching unit 404.

The matching unit 404 matches a two-dimensional picked-up image of the driver with the three-dimensional face structure data which constitutes the three-dimensional face model 410 (S32). In other words, the matching unit 404 performs model fitting and model tracking. Accordingly, the matching unit 404 specifies (estimates) a direction of the driver's face, and search for the eye positions on the three-dimensional face model 410 to specify (estimate) the positions.

Accordingly, in the embodiment, in the model fitting, an average face model created in a procedure illustrated in Fig. 7 is used as an initial state on the three-dimensional face model 410, which is a statistical face shape model, and characteristic points P of the model are positioned on corresponding parts of the face of the picked-up image to create a model M approximate to the face.

In the embodiment, in the model tracking, after the model M has been created in the model fitting, the model M is continuously matched with the face in the picked-up image of the driver picked up cyclically in S31. In the present embodiment, the model tracking is performed by using the template.

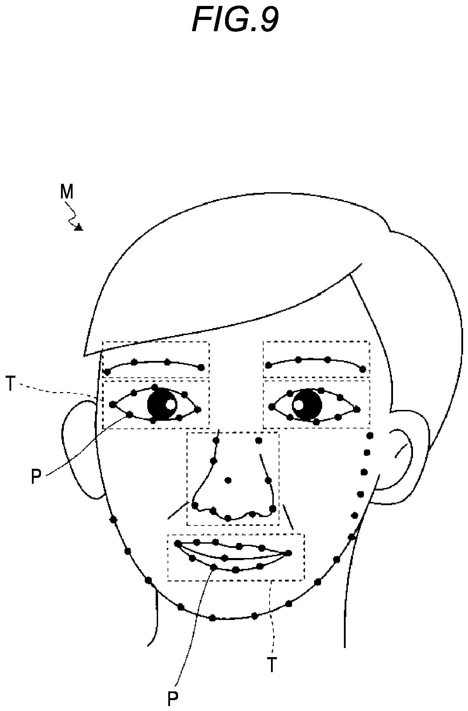

Fig. 9 is a schematic drawing illustrating a template T created for a model M of a face in the three-dimensional face model 410 of the embodiment. The template T has an area of a predetermined range including the characteristic points P of the model M in the images. For example, the template T includes an area including characteristic points P indicating eyebrows, an area including characteristic points P of eyes, an area including characteristic points P indicating a nose, an area including characteristic points P indicating a mouth, and an area including characteristic points P indicating an outline. The areas of the template T correspond to one or two or more characteristic points, and are mapped to coordinates of the characteristic points. In other words, if the position in the area that the template T has in the image is determined, a coordinate of the characteristic point P corresponding to the area can be calculated.

In the model tracking according to the embodiment, the template T determines the positions of the areas in the image, and determines an angle, a position, and a size of the model M in the image by using the positions of the areas. Subsequently, the determined angle, position, and size are applied to the model M, so that the model M can match the image. The position and the number of the areas that the template T has can be selected as desired as long as the model tracking is enabled.

Returning back to Fig. 8, since the eye positions are specified by the three-dimensional face model matched in S32, the shape parameter determination unit 405 creates the three-dimensional face model. The shape parameter determination unit 405 matches the two-dimensional picked-up image to the three-dimensional eye model (S33). In other words, the shape parameter determination unit 405 changes the shape parameter, and matches the eye shape corresponding to the shape parameter with the eye shape of the picked-up image. The shape parameter determination unit 405 specifies a shape parameter corresponding to the eye shape in the picked-up image from the three-dimensional face model 410.

Subsequently, the eye-opening calculating unit 406 calculates an eye-opening degree of the driver in the picked-up image (S34). The eye-opening degree indicates an eye-opening state of the driver, and in the present embodiment, the eye-opening degree corresponds to the distance between the upper and lower eyelids. A method of the eye-opening degree with the eye-opening calculating unit 406 used here is a known method such as calculating the eye-opening degree from pixels of the picked-up image. In the embodiment, the distance between the upper and lower eyelids is used as the eye-opening degree. However, the eye-opening degree is not limited thereto as long as it indicates the eye-opening state.

The downward gaze determination unit 407 determines whether the driver is in the eye-opening state or the eye-closing state on the basis of the shape parameter specified in S33 and the eye-opening degree calculated in S34. Specifically, the following process is performed.

The downward gaze determination unit 407 determines whether or not the eye-opening degree calculated in S34 is a predetermined close threshold value or smaller (S35). The close threshold value here corresponds to the maximum distance between upper and lower eyelids indicating the eye-closing state. Therefore, the eye-opening degree of the close threshold value or lower means that the eyes can be potentially closed. The close threshold value is determined in advance, and is memorized in the ROM 14b and the SSD 14f.

When the eye-opening degree is larger than the close threshold value (S35: No), the downward gaze determination unit 407 determines that driver is in the eye-opening state (S38), and the process terminates.

In contrast, when the eye-opening degree is the close threshold value or lower in S35 (S35: Yes), the downward gaze determination unit 407 determines whether or not the shape parameter specified in S33 is a predetermined downward gaze threshold value or lower (S36). The downward gaze threshold value is the maximum value of the shape parameter of the eye shape when the driver gases downward. Therefore, a case in which the shape parameter falls within a range not higher than the downward gaze threshold value means that the eye shape falls within a range of the eye shapes at the time of downward gaze. The downward gaze threshold value is determined in advance and is memorized in the ROM 14b and the SSD 14f. The downward gaze threshold value may be configured to be determined for each individual person. A configuration in which the downward gaze threshold value is changed depending on the circumstances is also applicable.

In the present embodiment, the eye shape falls within the range of a downward gaze when the shape parameter is the downward gaze threshold value or lower. The reason is because the shape parameter of the present embodiment is set to be smaller as the standard eye shape approaches a downward gaze as described above with reference to Fig. 6. Therefore, how to determine the downward gaze threshold value which corresponds to a border of the range of the downward gaze is different depending on how the shape parameter is set.

When the shape parameter is not higher than the downward gazes threshold value in S36 (S36: Yes), the downward gaze determination unit 407 determines that the driver gazes downward (S40). In other words, since the shape parameter is within the range of a downward gaze even though the eye-opening degree is not larger than the close threshold value and thus the distance between the upper and lower eyelids is small as a result of determination in S35, the downward gaze determination unit 407 determines that since the driver gazes downward, the distance between the upper and lower eyelids is small, and therefore, the driver is in the eye-opening state. Accordingly, the downward gaze and the eye-opening state of the driver can be detected further accurately. In this case, a state in which the driver is in the eye-opening state, but gazes downward for operating a smartphone or the like instead of looking forward during driving is conceivable. Therefore, the processing unit 408 causes an audio output device of the monitor device 11 to issue an alert 2 for making the driver look forward (S41), and the process terminates.

In contrast, when the shape parameter is larger than the downward gaze threshold value in S36 (S36: No), the downward gaze determination unit 407 determines that the driver is in the eye-closing state (S37). In other words, since the eye-opening degree is not larger than the eye-closing threshold value, and thus the distance between the upper and lower eyelids is small, as a result of determination in S35 and the shape parameter does not fall within a range of a downward gaze, the downward gaze determination unit 407 determines that the driver is closing the eyes. Accordingly, the state in which the driver' eyes are closed can be detected further accurately. In this case, since the driver is highly likely in drowsy driving, the processing unit 408 causes the audio output device of the monitor device 11 to output an alert 1 (S39). In the present embodiment, the alert 1 is output. However, the alert is not limited thereto as long as the alert gives warning to the driver to wake the driver up from the drowsy state.

In the present embodiment, since the eye shape in the driver's face is estimated (specified) by using not only the two-dimensional picked-up image, but also the three-dimensional face model, the result is not significantly affected by the relative position of the face in the direction vertical to the image pickup device 201 and the face direction. Therefore, according to the embodiment, since the eye shape can be estimated further accurately, whereby the states of the face and the eyes such as the state of opening/closing of the driver's eyes can be further accurately determined.

Hereinafter, another example in which a determination apparatus of an embodiment is mounted on a vehicle 1 will be described.

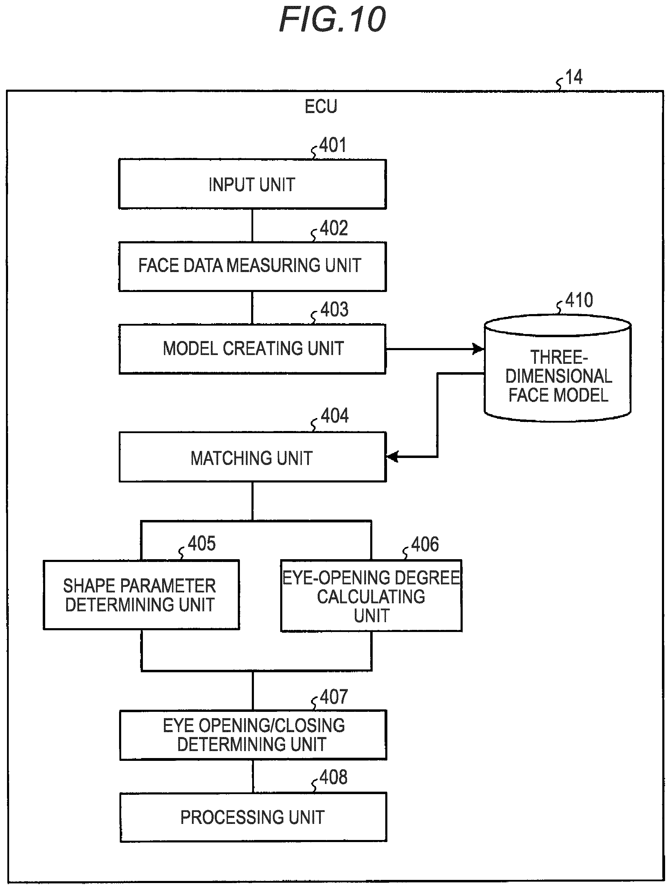

The basic configuration of the determination apparatus is the same as an eye opening/closing determination system 100 described above, and the different point is in that an eye opening/closing determination unit 407 in Fig. 10 is provided instead of the downward gaze determination unit 407 in Fig. 4. Steps of S360, S390, S400, and S410 in the flowchart (Fig. 8) in the determining process are different from Steps S36, S39, S40, and S41 in Fig. 8, respectively.

The different points will be mainly described below.

The ECU 14 of the another embodiment detects a direction of a driver's face and positions of the driver's eyes while tracking the driver's face, obtains an eye-opening degree, which is a distance between upper and lower eyelids of the driver, specifies a shape parameter corresponding to eye shape, and determines whether or not the driver is in a state of squinted eyes which is a state in which the state of narrowing the eyes in association with an expression change of the face such as a smile, and whether or not the driver opens the eyes on the basis of the eye-opening degree and the shape parameters by using the three-dimensional face model 410. Detailed description will be given below.

The ECU 14 of the another embodiment detects direction of the driver's face and the positions of the driver's eyes while tracking the driver's face, obtains an eye-opening degree, which is a distance between the upper and lower eyelids of the driver, specifies the shape parameter corresponding to the eye shape, and determines whether or not the driver is in a state of squinted eyes which is a state in which the state of narrowing the eyes in association with an expression change of the face such as a smile, and whether or not the driver opens the eyes on the basis of the eye-opening degree and the shape parameters by using the three-dimensional face model 410. Detailed description will be given below.

In addition to characteristic points P of the face (a face model M) as described above, change information is also registered in the three-dimensional face model 410. The change information is data that maps step-by-step change of the shape of the driver's eyes in association with the changes in expression of the face and actions of the driver to the shape parameters at each stage. Specifically, as the change states, a state in which the face has lack of expression and faces forward with opened eyes is defined as a standard state, and states such as a step-by-step change of the eye shape from the standard state until the expression is changed and shows smile, a step-by-step change of the eye shape from the standard state until the eyes are closed, and a step-by-step change of the eye shape from the standard state until the eyes gaze downward (downward gaze) are mapped to the shape parameters and are registered to the three-dimensional face model 410. Here, the smile is an example of the state of squinted eyes which corresponds to a state in which the eyes are narrowed in association with the expression change of the face.

A flowchart of the determination process will be described below.

Subsequently, an eye-opening calculating unit 406 calculates a degree of opening of the driver's eyes in a picked-up image (S34). The degree of opening of the eyes indicates the eye-opening state of the driver, and in the present embodiment, the eye-opening degree corresponds to the distance between the upper and lower eyelids. A method of calculating the eye-opening degree with the eye-opening calculating unit 406 used here is a known method such as calculating the eye-opening degree from pixels of the picked-up image. In the present embodiment, the distance between the upper and lower eyelids is used as the eye-opening degree. However, the eye-opening degree is not limited thereto as long as it indicates the eye-opening state.

An eye opening/closing determination unit 407 determines whether the driver is in the eye-opening state or the eye-closing state on the basis of the shape parameter specified in S33 and the eye-opening degree calculated in S34. Specifically, the following process is performed.

The eye opening/closing determination unit 407 determines whether or not the eye-opening degree calculated in S34 is a predetermined close threshold value or smaller (S35). The close threshold value here corresponds to the maximum distance between the upper and lower eyelids indicating the eye-closing state. Therefore, the eye-opening degree of the close threshold value or lower means that the eyes can be potentially closed. The close threshold value is determined in advance, and is memorized in the ROM 14b and the SSD 14f.

When the eye-opening degree is larger than the close threshold value (S35: No), the eye opening/closing determination unit 407 determines that the driver is in the eye-opening state (S38). The process is now terminated.

In contrast, when the eye-opening degree is not higher than the close threshold value in S35 (S35: Yes), the eye opening/closing determination unit 407 determines whether or not the shape parameter specified in S33 is not higher than a smile threshold value (S360). Here, the smile threshold value is the maximum value of the shape parameter of the eye shape with a smile. Therefore, the case where the shape parameter is in a range not higher than the smile threshold value means that the eye shape is within a range of the eye shape with a smile. The smile threshold value are determined in advance, and are memorized in the ROM 14b and the SSD 14f. The smile threshold value may be configured to be determined for each individual person. Alternatively, the smile threshold value may be configured to be changed depending on the circumstances.

Although the eye shape is determined to be within the range of a smile when the shape parameter is not higher than the smile threshold value in the present embodiment, the reason is that the shape parameter of the present embodiment is set to become smaller as the standard eye shape approaches a smile as described above with reference to Fig. 6. Therefore, how to determine the smile threshold value which corresponds to a border of the range of the smile is different depending on how the shape parameter is set.

When the shape parameter is not higher than the smile threshold value in S360 (S360: Yes), the eye opening/closing determination unit 407 determines that the driver is smiling (S400). In other words, since the shape parameter is within the range of the smile even though the eye-opening degree is not larger than the close threshold value and thus the distance between the upper and lower eyelids is small as a result of determination in S35, the eye opening/closing determination unit 407 determines that the driver is smiling and thus the distance between the upper and lower eyelids is small, and therefore, the driver is in the eye-opening state. Accordingly, the state in which the driver smiles or opens the eyes can be detected further accurately. The process is now terminated.

In contrast, when the shape parameter is larger than the smile threshold value in S360 (S360: No), the eye opening/closing determination unit 407 determines that the driver is in the eye-closing state (S370). In other words, since the eye-opening degree is not larger than the eye-closing threshold value and thus the distance between the upper and lower eyelids is small as a result of determination in S35, and the shape parameter does not fall within the range of the smile, the eye opening/closing determination unit 407 determines that the driver is closing the eyes. Accordingly, the state in which the driver closes the eyes can be detected further accurately. In this case, since the driver is highly likely in drowsy driving, a processing unit 408 outputs an alert from an audio output device of a monitor device 11 (S390). In the present embodiment, the alert is output. However, the alert is not limited thereto as long as the alert gives warning to the driver to wake the driver up from the drowsy driving state.

In this manner, according to the present embodiment, since the shape parameters corresponding to the changes in eye shape are registered to the three-dimensional face model 410, and expressions of the driver such as opening/closing of the eyes and a downward gaze are determined by using the shape parameters in addition to the eye-opening degree, estimation (specification) of expressions of the face or actions independent from a relative position or angle between a face and the image pickup device 201 is enabled. Therefore, according to the embodiment, the states of the driver's face and eyes such as opening/closing of the driver's eyes, and a state in which the driver gazes downward can be determined further accurately while avoiding erroneous determination.

Since the picked-up image is fitted to the three-dimensional face model in the embodiment disclosed here, the fitting is performed for the upper, lower, left, and right eyelids simultaneously. Therefore, detection of the states of the face and the eyes such as opening/closing of eyes, a state in which the driver gazes downward or a state in which the driver smiles can be detected robustly for a noise in the picked-up image compared with the related art in which curved lines of the upper, lower, left, and right eyelids are detected independently.

In the embodiment disclosed here, when the driver is determined to be in the state of gazing downward or closing the eyes, the processing unit 408 causes the audio output device of the monitor device 11 to output an alert as an example of the action of encouraging warning to the driver. Therefore, the occurrence or events in which the driver falls asleep at the wheel or drives with the eyes off the road is prevented.

In the embodiment, determination of the downward gaze is performed by registering the change in eye shape from the standard eye state to the downward gaze to the three-dimensional face model 410 together with the shape parameter. However, the three-dimensional face model 410, the shape parameter determination unit 405, the downward gaze determination unit 407, and the like may be configured in a manner of determination of items other than the downward gaze.

For example, a configuration in which the step-by-step changes of the eye shape from the standard eye state until a yawning are registered in the three-dimensional face model 410 together with the shape parameters, and the three-dimensional face model 410, the shape parameter determination unit 405, and the downward gaze determination unit 407 are configured to perform determination of the yawning is also applicable.

Alternatively, a configuration in which the step-by-step change of the eye shape from the standard eye state until a state of taking a good look at something are registered together with the shape parameters, and the three-dimensional face model 410, the shape parameter determination unit 405, and the downward gaze determination unit 407 are configured to determine the state in which the eyes are narrowed because of dazzling.

In the another embodiment, a smile is employed as an example of the state of squinted eyes which corresponds to a state in which the eyes are narrowed in association with the expression change of the face, the changes in eye shape from the standard eye state until a smile are registered in the three-dimensional face model 410 together with the shape parameters to determine the smile in eye opening/closing determination. However, the three-dimensional face model 410, the shape parameter determination unit 405, the eye opening/closing determination unit 407, and the like may be configured to determine the squinted eye state in which the eyes are narrowed in association with the expression change as expressions other than the smile.

Alternatively, the three-dimensional face model 410 may be configured so that the shape parameters are registered by being classified into differences in an individual and differences among individuals as described in the non-patent literature "Automatic Extraction of Eye Area Structure by Active Appearance Model Search" by Go Moriyama, Takeo Kanaide, and Jeffrey F. Cohn, Shinji Ozawa, "Meeting on Image Recognition and Understanding" (MIRU 2006)" and "Costen, N., Cootes, T.F., Taylor, C.J.: Compensating for ensemble-specificity effects when building facial models. Image and Vision Computing 20,673-682".

In the embodiment, determination of a downward gaze and determination of a smile are performed in order to achieve accurate determination that the driver opens or closes the eyes. However, the determination of the opening/closing of eyes is not limited thereto, and the three-dimensional face model 410, the shape parameter determination unit 405, and the downward gaze determination unit 407 and the like may be configured so as to employ determination of the downward gaze by using the method of the present embodiment in human communication such as interaction with an interface.

Although several embodiments have been described, these embodiments are intended for illustration only, and are not intended to limit the scope of this disclosure. These novel embodiments may be implemented in other various modes, and various omissions, replacements, and modifications may be made without departing from the gist of this disclosure. These embodiments and modifications thereof are included in the scope and gist of this disclosure, and are included in the invention described in the appended Claims and a range equivalent thereto.

The principles, preferred embodiment and mode of operation of the present invention have been described in the foregoing specification. However, the invention which is intended to be protected is not to be construed as limited to the particular embodiments disclosed. Further, the embodiments described herein are to be regarded as illustrative rather than restrictive. Variations and changes may be made by others, and equivalents employed, without departing from the spirit of the present invention. Accordingly, it is expressly intended that all such variations, changes and equivalents which fall within the spirit and scope of the present invention as defined in the claims, be embraced thereby.

A determination apparatus (14) includes: a memory (14f) memorizing a three-dimensional face model (410) in which a three-dimensional face shape and eye positions of a test subject, and change information mapping each state of change in eye shape to each shape parameter are registered; a matching unit (404) matching a picked-up image of a driver picked up by an image pickup device (201) with the three-dimensional face shape to specify the eye position; a shape parameter determination unit (405) changing the shape parameter to specify a shape parameter corresponding to the eye state in the picked-up image; an eye-opening degree calculating unit (406) calculating an eye-opening degree indicating a state of opening of the eye in the picked-up image; and a determination unit (407) determining a state of a face and an eye of the driver on the basis of the eye-opening degree and the specified shape parameter.

## Claims

1. A determination apparatus (14) comprising:

   a memory (14f) comprising memorized therein a three-dimensional face model (410) said model comprising registered therein a three-dimensional face shape of a driver eye positions of the driver and change information that maps step-by-step each change in the shape of the driver's eyes to an eye shape parameter, in association with changes in the state of the driver's face and eyes,

a matching unit (404) configured to match an image of the driver picked up by an image pickup device (201) with the three-dimensional face model to specify the direction of the driver's face and the eye positions;

a shape parameter determination unit (405) configured to specify a shape parameter corresponding to the shape of an eye in the picked-up image;

an eye-opening degree calculating unit (406) configured to calculate an eye-opening degree indicating a state of opening of the eye ; and

a determination unit (407) configured to determine the state of the face and eye of the driver on the basis of the specified eye-opening degree and the shape parameter, and the change information registered in the three dimensional model,

wherein the change information comprises information on a change from a standard state of the face and eye of the driver, in which the face has lack of expression and faces forward with the eyes open, to a state of gazing downward; and determination unit is configured to determine whether or not the driver is in a state of gazing downward on the basis of the specified eye-opening degree and shape parameter, and the change information registered in the three dimensional model.

2. The determination apparatus according to Claim 1, wherein the determination unit is configured to determine that the driver is in a state of gazing downwards, when the eye-opening degree is within a range indicating that the eyes are closed, and the shape parameter is associated with the state of gazing downwards.

3. The determination apparatus according to Claim 2, wherein
the determination unit is configured to determine that the driver is in the eye-closing state when the eye-opening degree is within the range indicating that the eyes are closed and the specified shape parameter is associated with the state of gazing downwards.

4. The determination apparatus according to Claim 2 or Claim 3, further including a processing unit (408) configured to perform an action of alerting the driver when the driver is determined to be in the eye-closing state and when the driver is determined to be in the downward gaze state.

5. The determination apparatus according to any one of Claims 1 to 4, further comprising:

a measuring unit (402) configured to obtain three-dimensional face structure data including the three-dimensional face shape and the eye positions on the basis of picked-up images of the driver, as well as change information that maps step-by-step each change in the shape of the driver's eyes to an eye shape parameter, in association with changes in the state of the driver's face and eyes, and

a model creating unit (403) configured to register the measured three-dimensional face structure data and the change information in the memory as the three-dimensional face model.

6. The determination apparatus according to any one of Claims 1 to 5, wherein the change information further comprises information on a change from the standard state to a state of squinted eyes; the determination unit (407) is further configured to determine opening and closing of the driver's eyes on the basis of whether or not the specified shape parameter is associated with a squinted eye state and of the eye-opening degree calculated by the eye-opening degree calculating unit.

7. The determination apparatus according to Claim 6, wherein
the squinted eye state corresponds to a state of smile, and
the determination unit is configured to determine whether or not the eye-opening degree falls within a range indicating an eye-closing state, determine whether or not the specified shape parameter is associated with the state of smile when the eye-opening degree falls within the range indicating the eye-closing state, and determine that the driver is in the eye-opening state when the shape parameter is associated with the state of smile.

8. The determination apparatus according to Claim 7, wherein
the determination unit is configured to determine that the driver is in the eye-closing state when the eye-opening degree is within the range indicating that the eyes are closed and when the specified shape parameter is not associated to a state of smile.

9. The determination apparatus according to Claim 8, further comprising:

a processing (408) unit configured to perform an action of alerting the driver when the driver is determined to be in the eye-closing state.

10. A determination method executed by a determination apparatus, the determination apparatus including: a memory (14f) configured to memorize a three-dimensional face model (410) in which a three-dimensional face shape of a test subject, eye positions of the test subject, and change information that maps step-by-step each change in the shape of the driver's eyes to an eye shape parameter, in association with changes in the state of the driver's face and eyes. are registered, the determination method comprising:

matching a picked-up image of a driver picked up by an image pickup device with the three-dimensional face shape to specify a face direction, and specifying the eye positions,
specifying a shape parameter corresponding to the shape of an eye in the picked-up image;
calculating an eye-opening degree indicating the state of opening of the eye in the picked-up image; and
determining the state of a face and an eye of the driver on the basis of the specified eye-opening degree and shape parameter, and the change information registered in the three dimensional model,
wherein the change information comprises information on a change from a standard state of the face and eye of the driver, in which the face has lack of expression and faces forward with the eyes open, to a state of gazing downward; and
the determining comprises determining whether or not the driver is in a state of gazing downward on the basis of the specified eye-opening degree and shape parameter, and the change information registered in the three dimensional model.

11. The determination method according to Claim 10, wherein
the change information further comprises information on a change from the standard state to a state of squinted eyes; and
the method further comprising:

determining the opening and closing of the driver's eyes on the basis of whether or not the specified shape parameter is associated to a squinted eye state even when the eye-opening degree indicates the eye-closing state.

**Patentansprüche**

1. Bestimmungsgerät (14) mit:

einem Speicher (14f) mit einem darin gespeicherten dreidimensionalen Gesichtsmodell (410), wobei das Modell eine darin verzeichnete drei-dimensionale Gesichtsform eines Fahrers, Augenpositionen des Fahrers, und Veränderungsinformationen umfasst, die Schritt für Schritt jede einzelne Veränderung in der Augenform des Fahrers auf einen Augenformparameter in Verbindung mit Veränderungen des Zustandes des Gesichts und der Augen des Fahrers abbilden,
einer Abgleichungseinheit (404), die dazu eingerichtet ist, ein durch eine Bildaufnahmevorrichtung (201) aufgenommenes Bild des Fahrers mit dem drei-dimensionalen Gesichtsmodell abzugleichen, um die Ausrichtung des Gesichtes und die Augenpositionen des Fahrers festzulegen;
einer Formparameterbestimmungseinheit (405), die dazu eingerichtet ist, in dem aufgenommenen Bild einen Formparameter, der der Form eines Auges entspricht, festzulegen;
einer Augenöffnungsgradberechnungseinheit (406), die dazu eingerichtet ist, einen Augenöffnungsgrad, der einen Zustand der Augenöffnung angibt, zu berechnen; und
einer Bestimmungseinheit (407), die dazu eingerichtet ist, den Zustand des Gesichtes und des Auges des Fahrers auf der Grundlage des festgelegten Augenöffnungsgrades und des Formparameters und der Veränderungsinformationen, die in dem drei-dimensionalen Modell verzeichnet sind, zu bestimmen,
wobei die Veränderungsinformationen Informationen über eine Veränderung von einem Standardzustand des Gesichtes und des Auges des Fahrers, in welchem es dem Gesicht an Ausdruck fehlt und es mit geöffneten Augen nach vorne gerichtet ist, zu einem Zustand des nach unten Starrens umfassen; und
die Bestimmungseinheit dazu eingerichtet ist, auf der Grundlage des festgelegten Augenöffnungsgrades und Formparameters des Auges und der Veränderungsinformationen, die in dem drei-dimensionalen Modell verzeichnet sind, zu bestimmen, ob der Fahrer in einem Zustand des nach unten Starrens ist oder nicht.

2. Bestimmungsgerät nach Anspruch 1, wobei
die Bestimmungseinheit dazu eingerichtet ist, zu bestimmen, dass der Fahrer in einem Zustand des nach unten Starrens ist, wenn der Augenöffnungsgrad innerhalb eines Bereiches ist, der angibt, dass die Augen geschlossen sind, und der Formparameter mit dem Zustand des nach unten Starrens in Verbindung steht.

3. Bestimmungsgerät nach Anspruch 2, wobei

die Bestimmungseinheit dazu eingerichtet ist, zu bestimmen, dass der Fahrer in einem Zustand des Augenschließens ist, wenn der Augenöffnungsgrad in einem Bereich ist, der angibt, dass die Augen geschlossen sind, und der festgelegte Formparameter mit dem Zustand des nach unten Starrens in Verbindung steht.

4. Bestimmungsgerät nach Anspruch 2 oder Anspruch 3, ferner mit einer Verarbeitungseinheit (408), die dazu eingerichtet ist, eine Maßnahme zur Alarmierung des Fahrers durchzuführen, wenn bestimmt wird, dass der Fahrer im Zustand des Augenschließens ist, und, wenn bestimmt wird, dass der Fahrer im Zustand des nach unten Starrens ist.

5. Bestimmungsgerät nach einem der Ansprüche 1 bis 4, ferner mit:

   einer Messeinheit (402), die dazu eingerichtet ist, drei-dimensionale Gesichtsstrukturdaten zu gewinnen, die die drei-dimensionale Gesichtsform und die Augenpositionen auf der Grundlage der aufgenommenen Bilder des Fahrers enthalten, wie auch Veränderungsinformationen, die Schritt für Schritt jede einzelne Veränderung in der Augenform des Fahrers auf einen Augenformparameter in Verbindung mit Veränderungen des Zustandes des Gesichts und der Augen abbilden, und
   eine Modellerzeugungseinheit (403), die dazu eingerichtet ist, die gemessenen drei-dimensionalen Gesichtsstrukturdaten und die Informationen zur Veränderung im Speicher als das drei-dimensionale Gesichtsmodell zu verzeichnen.

6. Bestimmungsgerät nach einem der Ansprüche 1 bis 5,
   wobei
   die Veränderungsinformationen ferner Informationen über eine Veränderung von dem Standardzustand zu einem Zustand eines Augenzusammenkneifens umfassen;
   die Bestimmungseinheit (407) ferner dazu eingerichtet ist, das Öffnen und Schließen der Augen des Fahrers auf der Grundlage, ob der festgelegte Formparameter in Verbindung mit einem Zustand eines Augenzusammenkneifens steht oder nicht, und des Augenöffnungsgrades, der durch die Einheit zur Berechnung des Augenöffnungsgrades berechnet wird, zu bestimmen.

7. Bestimmungsgerät nach Anspruch 6, wobei der Zustand eines zusammengekniffenen Auges einem Zustand des Lächelns entspricht, und die Bestimmungseinheit dazu eingerichtet ist, zu bestimmen, ob der Augenöffnungsgrad in einen Bereich fällt, der einen Zustand des Augenschließens anzeigt, oder nicht, und festzustellen, ob der festgelegte Formparameter mit dem Zustand des Lächelns in Verbindung steht, wenn der Augenöffnungsgrad in einen Bereich fällt, der den Zustand des Augenschließens anzeigt, und festzustellen, dass der Fahrer in dem Zustand der Augenöffnung ist, wenn der Formparameter mit dem Zustand des Lächelns in Verbindung steht.

8. Bestimmungsgerät nach Anspruch 7, wobei die Bestimmungseinheit dazu eingerichtet ist, zu bestimmen, dass der Fahrer in dem Zustand des Augenschließens ist, wenn der Augenöffnungsgrad in einem Bereich ist, der anzeigt, dass die Augen geschlossen sind, und wenn der festgelegte Formparameter nicht in Verbindung mit dem Zustand des Lächelns steht.

9. Bestimmungsgerät nach Anspruch 8, ferner mit:

   einer Verarbeitungseinheit (408), die dazu eingerichtet ist, eine Maßnahme zur Alarmierung des Fahrers durchzuführen, wenn bestimmt wird, dass der Fahrer in einem Zustand des Augenschließens ist.

10. Bestimmungsverfahren, das durch ein Bestimmungsgerät durchgeführt wird, wobei das Bestimmungsgerät umfasst:

   einen Speicher (14f), der dazu eingerichtet ist, ein drei-dimensionales Gesichtsmodell (410) zu speichern, in welchem eine drei-dimensionale Gesichtsform eines Testsubjektes, Augenpositionen des Testsubjektes, und Veränderungsinformationen verzeichnet werden, die Schritt für Schritt jede einzelne Veränderung in der Augenform des Fahrers auf einen Augenformparameter in Verbindung mit Veränderungen des Zustandes des Gesichts und der Augen abbilden, das Bestimmungsverfahren umfasst:

      Abgleichen eines durch eine Bildaufnahmevorrichtung aufgenommenen Bildes eines Fahrers mit der drei-dimensionalen Gesichtsform, um eine Ausrichtung des Gesichts festzustellen, und
      Feststellen der Augenpositionen;
      Feststellen eines Formparameters, der der Form eines Auges in dem aufgenommenen Bild entspricht;
      Berechnen eines Augenöffnungsgrades, der dem Zustand der Augenöffnung in dem aufgenommenen Bild entspricht; und
      Bestimmen des Zustandes eines Gesichtes und eines Auges des Fahrers auf der Grundlage des festgelegten Augenöffnungsgrades und des festgelegten Formparameters, und der in dem drei-dimensionalen Modell verzeichneten Veränderungsinformationen; wobei

die Veränderungsinformationen Informationen über eine Veränderung von einem Standardzustand des Gesichtes und Auges des Fahrers, in welchen es dem Gesicht an Ausdruck fehlt und es mit geöffneten Augen nach vorne gerichtet ist, zu einem Zustand des nach unten Starrens umfassen, und das Bestimmen ein Bestimmen umfasst, ob der Fahrer in einem Zustand des nach unten Starrens ist oder nicht auf der Grundlage des festgelegten Augenöffnungsgrades und des Formparameters, und der in dem dreidimensionalen Modell verzeichneten Veränderungsinformationen.

11. Bestimmungsverfahren nach Anspruch 10, wobei die Veränderungsinformationen ferner Informationen zu einer Veränderung von dem Standardzustand zu einem Zustand eines Augenzusammenkneifens umfassen; und das Verfahren ferner umfasst:

Bestimmen des Öffnens und Schließens der Augen des Fahrers auf der Grundlage, ob der festgelegte Formparameter in Verbindung mit einem Zustand eines Augenzusammenkneifens steht oder nicht, selbst wenn der Augenöffnungsgrad auf den Zustand des Augenschließens hinweist.

**Revendications**

1. Appareil de détermination (14) comprenant :

une mémoire (14f) comprenant, mémorisé dans celle-ci, un modèle de visage tridimensionnel (410), ledit modèle comprenant, enregistrées dans celui-ci, une forme de visage tridimensionnelle d'un conducteur, des positions des yeux du conducteur, et des informations de changement qui mappent pas par pas chaque changement de la forme des yeux du conducteur vers un paramètre de forme des yeux, en association avec des changements de l'état du visage et des yeux du conducteur, une unité de mise en correspondance (404) configurée pour mettre en correspondance une image du conducteur prise par un dispositif de prise d'image (201) avec le modèle de visage tridimensionnel pour spécifier la direction du visage du conducteur et les positions des yeux ; une unité de détermination de paramètre de forme (405) configurée pour spécifier un paramètre de forme correspondant à la forme d'un oeil dans l'image prise ; une unité de calcul de degré d'ouverture des yeux (406) configurée pour calculer un degré d'ouverture des yeux indiquant un état d'ouverture de l'oeil ; et une unité de détermination (407) configurée pour déterminer l'état du visage et de l'oeil du conducteur sur la base du degré d'ouverture des yeux et d'un paramètre de forme spécifiés, et des informations de changement enregistrées dans le modèle tridimensionnel, dans lequel les informations de changement comprennent des informations concernant un changement d'un état standard du visage et de l'oeil du conducteur, dans lequel le visage manque d'expression et est orienté vers l'avant avec les yeux ouverts, vers un état de regard vers le bas ; et l'unité de détermination est configurée pour déterminer si, oui ou non, le conducteur est dans un état de regard vers le bas sur la base d'un degré d'ouverture des yeux et d'un paramètre de forme spécifiés, et des informations de changement enregistrées dans le modèle tridimensionnel.

2. Appareil de détermination selon la revendication 1, dans lequel l'unité de détermination est configurée pour déterminer que le conducteur est dans un état de regard vers le bas, lorsque le degré d'ouverture des yeux est dans une plage indiquant que les yeux sont fermés, et que le paramètre de forme est associé à l'état de regard vers le bas.

3. Appareil de détermination selon la revendication 2, dans lequel l'unité de détermination est configurée pour déterminer que le conducteur est dans l'état de fermeture des yeux lorsque le degré d'ouverture des yeux est dans la plage indiquant que les yeux sont fermés et que le paramètre de forme spécifié est associé à l'état de regard vers le bas.

4. Appareil de détermination selon la revendication 2 ou la revendication 3, comprenant en outre une unité de traitement (408) configurée pour effectuer une action d'alerte du conducteur lorsqu'il est déterminé que le conducteur est dans un état de fermeture des yeux et lorsqu'il est déterminé que le conducteur est dans un état de regard vers le bas.

5. Appareil de détermination selon l'une quelconque des revendications 1 à 4, comprenant en outre :

une unité de mesure (402) configurée pour obtenir des données de structure de visage tridimensionnelle comprenant la forme de visage tridimensionnelle et les positions des yeux sur la base des images prises du conducteur, ainsi que des informations de changement qui mappent pas par pas chaque changement de la forme des yeux du conducteur vers un paramètre de forme des yeux, en association avec des changements de l'état du visage et des yeux du conducteur, et

une unité de création de modèle (403) configurée pour enregistrer les données de structure de visage tridimensionnelle mesurées et les informations de changement dans la mémoire en tant que modèle de visage tridimensionnel.

**6.** Appareil de détermination selon l'une quelconque des revendications 1 à 5, dans lequel

les informations de changement comprennent en outre des informations concernant un changement de l'état standard vers un état des yeux plissés ;

l'unité de détermination (407) est en outre configurée pour déterminer une ouverture et une fermeture des yeux du conducteur selon que le paramètre de forme spécifié est associé ou non à un état des yeux plissés et sur la base du degré d'ouverture des yeux calculé par l'unité de calcul de degré d'ouverture des yeux.

**7.** Appareil de détermination selon la revendication 6, dans lequel

l'état des yeux plissés correspond à un état de sourire, et

l'unité de détermination est configurée pour déterminer si, oui ou non, le degré d'ouverture des yeux tombe dans une plage indiquant un état de fermeture des yeux, pour déterminer si, oui ou non, le paramètre de forme spécifié est associé à l'état de sourire lorsque le degré d'ouverture des yeux tombe dans la plage indiquant l'état de fermeture des yeux, et pour déterminer que le conducteur est dans l'état d'ouverture des yeux lorsque le paramètre de forme est associé à l'état de sourire.

**8.** Appareil de détermination selon la revendication 7, dans lequel

l'unité de détermination est configurée pour déterminer que le conducteur est dans l'état de fermeture des yeux lorsque le degré d'ouverture des yeux est dans la plage indiquant que les yeux sont fermés et lorsque le paramètre de forme spécifié n'est pas associé à un état de sourire.

**9.** Appareil de détermination selon la revendication 8, comprenant en outre :

une unité de traitement (408) configurée pour effectuer une action d'alerte du conducteur lorsqu'il est déterminé que le conducteur est dans l'état de fermeture des yeux.

**10.** Procédé de détermination exécuté par un appareil de détermination, l'appareil de détermination comprenant : une mémoire (14f) configurée pour mémoriser un modèle de visage tridimensionnel (410) dans lequel une forme de visage tridimensionnelle d'un sujet de test, des positions des yeux du sujet de test, et des informations de changement qui mappent pas par pas chaque changement de la forme des yeux du conducteur vers un paramètre de forme des yeux, en association avec des changements de l'état du visage et des yeux du conducteur, sont enregistrées, le procédé de détermination comprenant :

la mise en correspondance d'une image prise d'un conducteur prise par un dispositif de prise d'image avec la forme de visage tridimensionnelle pour spécifier une direction de visage, et la spécification des positions des yeux, la spécification d'un paramètre de forme correspondant à la forme d'un oeil dans l'image prise ; le calcul d'un degré d'ouverture des yeux indiquant l'état d'ouverture de l'oeil dans l'image prise ; et

la détermination de l'état d'un visage et d'un oeil du conducteur sur la base du degré d'ouverture des yeux et du paramètre de forme spécifiés, et des informations de changement enregistrées dans le modèle tridimensionnel, dans lequel les informations de changement comprennent des informations concernant un changement par rapport à un état standard du visage et d'un oeil du conducteur, dans lequel le visage manque d'expression et est orienté vers l'avant avec les yeux ouverts, vers un état de regard vers le bas ; et

la détermination comprend la détermination si, oui ou non, le conducteur est dans un état de regard vers le bas sur la base du degré d'ouverture des yeux et du paramètre de forme spécifiés, et des informations de changement enregistrées dans le modèle tridimensionnel.

**11.** Procédé de détermination selon la revendication 10, dans lequel

les informations de changement comprennent en outre des informations concernant un changement de l'état standard vers un état des yeux plissés ; et le procédé comprenant en outre :

la détermination de l'ouverture et de la fermeture des yeux du conducteur selon que le paramètre de forme spécifié est associé ou non à un état des yeux plissés même lorsque le degré d'ouverture des yeux indique l'état de fermeture des yeux.

# FIG.1

# FIG.2

# FIG.3

EP 3 033 999 B1

# FIG.4

14

ECU

401

INPUT UNIT

402

FACE DATA MEASURING UNIT

403

MODEL CREATING UNIT

410

THREE-DIMENSIONAL FACE MODEL

404

MATCHING UNIT

405

SHAPE PARAMETER DETERMINING UNIT

406

EYE-OPENING DEGREE CALCULATING UNIT

407

DOWNWARD GAZE DETERMINATION UNIT

408

PROCESSING UNIT

# FIG.5

# FIG.6

| SHAPE PARAMETER 1 | STANDARD → CLOSE | SHAPE PARAMETER 2 | STANDARD → SMILE | SHAPE PARAMETER 3 | STANDARD → DOWNWARD GAZE | ... |
|---|---|---|---|---|---|---|
| 100a | | 100a | | 100a | | ... |
| 70a | | 80b | | 60c | | ... |
| 50a | | 40b | | 40c | | ... |
| ⋮ | | ⋮ | | ⋮ | | |
| 20a | | 10b | | 20c | | ... |

# FIG.7

```
        ┌─────────────────┐
        │      START      │
        └─────────────────┘
                 │
                 ▼
┌───────────────────────────────────────────┐
│   PICKE-UP IMAGE OF FACE OF TEST SUBJECT   │──── S11
└───────────────────────────────────────────┘
                 │
                 ▼
┌───────────────────────────────────────────┐
│  MEASURE THREE-DIMENSIONAL FACE STRUCTURE DATA │──── S12
└───────────────────────────────────────────┘
                 │
                 ▼
┌───────────────────────────────────────────┐
│      CREATE THREE-DIMENSIONAL FACE MODEL   │──── S13
└───────────────────────────────────────────┘
                 │
                 ▼
        ┌─────────────────┐
        │       END       │
        └─────────────────┘
```

# FIG.8

START

PICKUP IMAGE OF DRIVER'S FACE —S31

MATCH TWO-DIMENSIONAL PICKED-UP IMAGE WITH THREE-DIMENSIONAL FACE MODEL (SPECIFY FACE DIRECTION, AND SEARCH FOR AND SPECIFY EYE POSITIONS —S32

MATCH TWO-DIMENSIONAL PICKED-UP IMAGE WITH THREE-DIMENSIONAL EYE-MODEL (CHANGE SHAPE PARMETER AND SPECIFY SHAPE PARAMETER) —S33

CALCULATE EYE-OPENING DEGREE —S34

S35
EYE-OPENING DEGREE ≦ CLOSE THRESHOLD VALUE ? — NO

YES

S36
SHAPE PARAMETER ≦ DOWNWARD GAZE THRESHOLD VALUE ? — YES

NO —S37

DETERMINE AS OPENED EYE —S38

DETERMINE AS CLOSED EYE —S37

DETERMINE AS DOWNWARD GAZE —S40

OUTPUT ALERT 1 —S39

OUTPUT ALERT 2 —S41

END

# FIG.9

# FIG.10

14

ECU

401

INPUT UNIT

402

FACE DATA MEASURING UNIT

403

MODEL CREATING UNIT

410

THREE-DIMENSIONAL FACE MODEL

404

MATCHING UNIT

405

SHAPE PARAMETER DETERMINING UNIT

406

EYE-OPENING DEGREE CALCULATING UNIT

407

EYE OPENING/CLOSING DETERMINING UNIT

408

PROCESSING UNIT

# FIG.11

```
                    ┌─────────────┐
                    │    START    │
                    └──────┬──────┘
                           │
         ┌─────────────────────────────────────┐
         │  PICKUP IMAGE OF DRIVER'S FACE       │──S31
         └─────────────────┬───────────────────┘
                           │
         ┌─────────────────────────────────────┐
         │  MATCH TWO-DIMENSIONAL PICKED-       │
         │  UP IMAGE WITH THREE-DIMENSIONAL     │
         │  FACE MODEL (SPECIFY FACE            │──S32
         │  DIRECTION, AND SEARCH FOR           │
         │  AND SPECIFY EYE POSITIONS           │
         └─────────────────┬───────────────────┘
                           │
         ┌─────────────────────────────────────┐
         │  MATCH TWO-DIMENTIONAL PICKED-       │
         │  UP IMAGE WITH THREE-DIMENTIONAL     │
         │  EYE-MODEL (CHANGE SHAPE             │──S33
         │  PARMETER AND SPECIFY                │
         │  SHAPE PARAMETER)                    │
         └─────────────────┬───────────────────┘
                           │
         ┌─────────────────────────────────────┐
         │  CALCULATE EYE-OPENING DEGREE        │──S34
         └─────────────────┬───────────────────┘
```

EYE-OPENING DEGREE ≦ CLOSE THRESHOLD VALUE?  —S35

NO → DETERMINE AS OPENED EYE —S38

YES

SHAPE PARAMETER ≦ SMILE THRESHOLD VALUE?  —S360

YES → DETERMINE AS SMILE —S400

NO

DETERMINE AS CLOSED EYE —S370

OUTPUT ALERT —S390

END

## REFERENCES CITED IN THE DESCRIPTION

**Patent documents cited in the description**

- JP 2008165348 A
- JP 2004192551 A
- JP 2008171065 A
- JP 2008167806 A

- US 2011235919 A1
- US 2014140577 A1
- JP 2011128966 A

**Non-patent literature cited in the description**

- **TAKAHIRO ISHIKAWA et al.** Passive Driver Gaze Tracking With Active Appearance Models. *IEEE Transactions on Intelligent Transportation Systems,* 01 October 2004
- **GO MORIYAMA ; TAKEO KANAIDE ; JEFFREY F. COHN ; SHINJI OZAWA.** Automatic Extraction of Eye Area Structure by Active Appearance Model Search. *Meeting on Image Recognition and Understanding,* 2006

- **COSTEN, N. ; COOTES, T.F. ; TAYLOR, C.J.** Compensating for ensemble-specificity effects when building facial models. *Image and Vision Computing,* vol. 20, 673-682